(19) Europäisches Patentamt
European Patent Office
Office européen des brevets

(11) Veröffentlichungsnummer: **0 498 380 A1**

# (12) EUROPÄISCHE PATENTANMELDUNG

(21) Anmeldenummer: **92101854.5**

(51) Int. Cl.[5]: **C07D 213/69**, A61K 31/44

(22) Anmeldetag: **05.02.92**

(30) Priorität: **08.02.91 CH 401/91**

(43) Veröffentlichungstag der Anmeldung:
**12.08.92 Patentblatt 92/33**

(84) Benannte Vertragsstaaten:
**PT**

(71) Anmelder: **BYK GULDEN LOMBERG CHEMISCHE FABRIK GMBH**
**Postfach 100310, Byk-Guldenstrasse 2**
**W-7750 Konstanz(DE)**

(72) Erfinder: **Beller, Klaus-Dieter**
**Franz-Moser-Strasse 5**
**W-7750 Konstanz 16(DE)**
Erfinder: **Hummel, Rolf-Peter**
**Von-Lassberg-Strasse 19a**
**W-7758 Meersburg(DE)**
Erfinder: **Kley, Hans-Peter**
**Im Weinberg 3b**
**W-7753 Allensbach(DE)**
Erfinder: **Kohl, Bernhard**
**Zum Brühl 9**
**W-7750 Konstanz 18(DE)**

(54) **Komplexbildner.**

(57) Komplexbildner der allgemeinen Formel I, worin

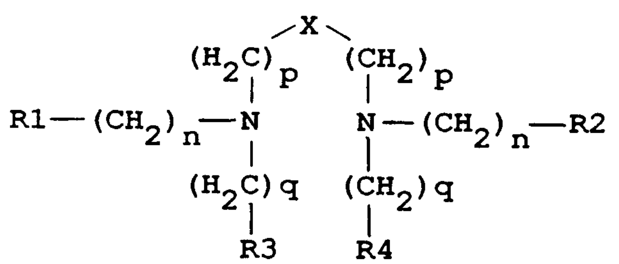

(I),

| | |
|---|---|
| R1, R2, R3 und R4 | gleich oder verschieden sind und für H, COOH, C(O)NHR5, wobei |
| R5 | H und C1-C4-Alkyl bedeutet, $PO_3H_2$, $SO_3H$ und den Rest 3-Hydroxy-1-(R6)-4-[1H]pyridon-2-yl, wobei |
| R6 | H und C1-C4-Alkyl bedeutet, stehen, mit der Maßgabe, daß mindestens einer der Reste R1, R2, R3 und R4 die Bedeutung 3-Hydroxy-1-(R6)-4-[1H]pyridon-2-yl mit R6 = H und C1-C4-Alkyl hat, |
| X | für eine Einfachbindung, $(CH_2)_m$ und NR7, wobei |
| R7 | H, C1-C4-Alkyl und $(CH_2)_nR1$ und |
| m | 1 und 2 bedeuten, |
| n,q | für ganze Zahlen von 0 bis 2, wobei n und q gleich oder verschieden sind und deren Summe mindestens 1 beträgt, und |
| p | für eine ganze Zahl von 1 bis 3, aber nicht für 1, wenn X die Bedeutung NR7 hat, |

stehen und deren Salze mit organischen und anorganischen Basen und Säuren, eignen sich zur Herstellung von paramagnetischen Komplexen, die sich vorteilhaft als Kontrastmittel für die MR-Diagnostik einsetzen lassen.

EP 0 498 380 A1

## Technisches Gebiet

Die Erfindung bezieht sich auf neue Komplexbildner.

## Stand der Technik

Es ist bekannt, in der MR-Diagnostik paramagnetische Metallkomplexverbindungen zur Kontrastverstärkung einzusetzen. Lösungen eines Gadolinium(III)-Komplexes mit dem Komplexbildner Diethylentriaminpentaessigsäure [Gd(DTPA)] haben sich als MR-Kontrastmittel bereits in der Praxis bewährt. Um die Verwendung des in freier Form hochtoxischen Gadoliniums zu vermeiden und um eine Spezifität zur Darstellung bestimmter Organe zu erreichen, wurden verschiedene Komplexe anderer paramagnetischer Metallionen vorgeschlagen. Die EP-A-0235361 schlägt u.a. vor, einen Eisen(III)-Komplex der bekannten Trihydroxamsäure Desferrioxamin (Desferal®) als MR-Kontrastmittel insbesondere zur Darstellung des Urogenitaltrakts einzusetzen.

Bei klinischen Untersuchungen hat sich dieser Komplex im Hinblick auf die kontrastverstärkende Wirkung als sehr gut erwiesen. Als nachteilig an diesem Komplex wird jedoch dessen unerwünschte blutdruck- und herzfrequenzsenkende Wirkung (W. L. Niedrach et al., Investigative Radiology, 23 [1988] 687 - 691) empfunden.

Aus G. J. Kontoghiorghes, Inorg. Chim. Acta 135 [1987] 145 - 150, und C. Hershko, Brit. Med. J. 296 - [1988] 1081, ist bekannt, daß bestimmte Hydroxypyridone geeignet sind, um mit Eisen(III)ionen stabile Komplexe zu bilden und daher oral verabreicht zur Eliminierung von überschüssigem Eisen aus dem menschlichen Körper eingesetzt werden können.

Von M. Streater et al., J. Med. Chem. 33 [1990] 1749 - 1755, werden die sechszähnigen Komplexbildner N,N,N-Tris-[2-(3-hydroxy-2-oxo-1,2-dihydropyridin-1-yl)acetamido]alkylamine und deren Komplexe mit Eisen(III) beschrieben. Die Komplexbildner sollen sich zur Entfernung von Eisen aus eisenüberladenen Patienten eignen.

Aus der EP-A-0290047 sind Mangan(II)-Komplexe mit N,N'-Bis-(pyridoxal-5-phosphat)-$\alpha$,$\omega$-(alkylen)-diamin-N,N'-essigsäuren bekannt, die sich als MR-Kontrastmittel eignen sollen.

Aufgabe der vorliegenden Erfindung ist es, neue Komplexbildner zur Verfügung zu stellen, deren Komplexe mit paramagnetischen Metallionen sich durch eine hohe Komplexstabilität, eine gute Wasserlöslichkeit bei physiologischen pH-Werten, eine starke Relaxivität und durch eine ausgeprägte Organspezifität verbunden mit verringerten Nebenwirkungen und schneller Ausscheidung auszeichnen.

## Beschreibung der Erfindung

Gegenstand der Erfindung sind Komplexbildner der allgemeinen Formel I,

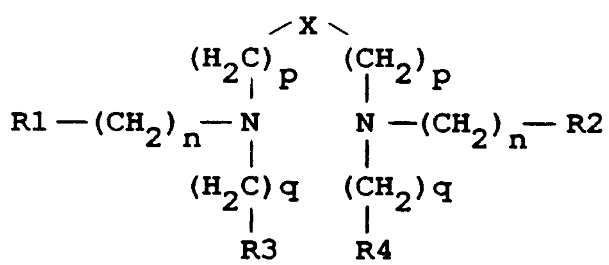

(I),

worin

R1, R2, R3 und R4 gleich oder verschieden sind und für H, COOH, C(O)NHR5,
wobei
R5 H und C1-C4-Alkyl bedeutet,
$PO_3H_2$, $SO_3H$ und den Rest 3-Hydroxy-1-(R6)-4-[1H]pyridon-2-yl,
wobei
R6 H und C1-C4-Alkyl bedeutet,
stehen, mit der Maßgabe, daß mindestens einer der Reste R1, R2, R3 und R4 die Bedeutung 3-Hydroxy-1-(R6)-4-[1H]pyridon-2-yl mit R6 = H und C1-C4-Alkyl hat,
X für eine Einfachbindung, $(CH_2)_m$ und NR7,
wobei

| | |
|---|---|
| R7 | H, C1-C4-Alkyl und $(CH_2)_nR1$ und |
| m | 1 und 2 bedeuten, |
| n,q | für ganze Zahlen von 0 bis 2, wobei n und q gleich oder verschieden sind und deren Summe mindestens 1 beträgt, und |
| p | für eine ganze Zahl von 1 bis 3, aber nicht für 1, wenn X die Bedeutung NR7 hat, |

stehen und deren Salze mit organischen und anorganischen Basen und Säuren.

Ein bevorzugter Gegenstand der Erfindung sind Komplexbildner der vorstehenden allgemeinen Formel I, wobei

| | |
|---|---|
| R1, R2, R3 und R4 | gleich oder verschieden sind und für H, COOH, C(O)NHR5, wobei |
| R5 | H, Methyl und Ethyl bedeutet, und den Rest 3-Hydroxy-4-[1H]-pyridon-2-yl stehen, mit der Maßgabe, daß mindestens einer der Reste R1, R2, R3 und R4 für die Bedeutung 3-Hydroxy-4-[1H]pyridon-2-yl steht, |
| X | für eine Einfachbindung, $CH_2$ und NR7, wobei |
| R7 | H und $(CH_2)_nR1$ bedeutet, |
| n,q | für 1 und |
| p | für 1 und 2, aber nicht für 1, wenn X die Bedeutung NR7 hat, und nicht für 2, wenn X die Bedeutung Einfachbindung hat, |

stehen, und deren Salze mit organischen und anorganischen Basen und Säuren.

Ein besonders bevorzugter Gegenstand der Erfindung sind Komplexbildner der vorstehenden allgemeinen Formel I, worin

| | |
|---|---|
| R1, R2, R3 und R4 | gleich oder verschieden sind und für H, COOH und den Rest 3-Hydroxy-4-[1H]-pyridon-2-yl stehen, mit der Maßgabe, daß mindestens einer der Reste R1, R2, R3 und R4 für den Rest 3-Hydroxy-4-[1H]pyridon-2-yl steht, |
| X | für eine Einfachbindung, |
| n | für 1, |
| q | für 0 und 1 und |
| p | für 1 |

stehen, und deren Salze mit organischen und anorganischen Basen und Säuren.

Ein gleichermaßen bevorzugter Gegenstand der Erfindung sind Komplexbildner der vorstehenden allgemeinen Formel I, worin

| | |
|---|---|
| R1, R2, R3 und R4 | gleich oder verschieden sind und für H, COOH und den Rest 3-Hydroxy-4-[1H]-pyridon-2-yl stehen, mit der Maßgabe, daß mindestens einer der Reste R1, R2, R3 und R4 für den Rest 3-Hydroxy-4-[1H]pyridon-2-yl steht, |
| X | für NR7, wobei R7 $(CH_2)_nR1$ bedeutet, |
| n | für 1, |
| q | für 0 und 1 und |
| p | für 2 |

stehen, und deren Salze mit organischen und anorganischen Basen und Säuren.

Besonders bevorzugte Komplexbildner sind N,N'-Bis-(3-hydroxy-4-[1H]pyridon-2-yl-methyl)-ethylendiamin-N,N'-diessigsäure, N,N'-Bis-(3-hydroxy-4-[1H]pyridon-2-yl-methyl)ethylendiamin-monoessigsäure, N,N'-Bis-(3-hydroxy-4-[1H]-pyridon-2-yl-methyl)ethylendiamin, N,N''-Bis-(3-hydroxy-4-[1H]pyridon-2-yl-methyl)diethylentriamin-N,N',N''-triessigsäure, N-(3-Hydroxy-4-[1H]pyridon-2-yl-methyl)ethylendiamin-N,N',N'-triessigsäure, N'-(3-Hydroxy-4-[1H]pyridon-2-yl-methyl)-diethylentriamin-N,N,N'',N''-tetraessigsäure und N-(3-Hydroxy-4-[1H]pyridon-2-yl-methyl)ethylendiamin-N,N'-diessigsäure und deren Salze mit organischen und anorganischen Basen und Säuren. Bevorzugt sind die Hydrochloride und Hydrobromide.

Gegenstand der Erfindung ist auch ein Verfahren zur Herstellung der Komplexbildner der allgemeinen Formel I, das dadurch gekennzeichnet ist, daß man eine Verbindung der allgemeinen Formel I, bei der die 3-Hydroxy-4-[1H]-pyridon-2-yl-Reste durch 3,4-Dihydroxypyridin-2-yl-Reste, deren Hydroxygruppen durch für Hydroxygruppen übliche Schutzgruppen, vorzugsweise Benzyl und Methyl, geschützt sind, ersetzt sind, unter die Schutzgruppen abspaltenden Bedingungen umsetzt und gewünschtenfalls die 3-Hydroxy-4-[1H]-pyridon-2-yl-Reste in 1-Stellung C1-C4-alkyliert.

Die Herstellung der erfindungsgemäßen Komplexbildner kann ausgehend von den entsprechenden 3,4-Bis-hydroxypyridin-2-aldehyden erfolgen, deren Hydroxygruppen durch geeignete Schutzgruppen, z.B. Methyl oder Benzyl, geschützt sind. Durch Umsetzung nach den für die Kondensierung von Aldehydgruppen mit Aminen üblichen Bedingungen mit den entsprechenden Alkylendiaminen bzw. Dialkylentriaminen und anschließende Reduktion werden OH-geschützte 3,4-Bis-hydroxy-2-pyridyl-amine erhalten. Diese werden mit entsprechenden ω-Halogen-, vorzugsweise ω-Brom-, $-(CH_2)_n$-R1-Verbindungen umgesetzt. Zur Herstellung von Komplexbildnern, bei denen R1 die Bedeutung COOH hat, können hierbei auch ω-Halogen-$(CH_2)_n$-R1-Verbindungen zum Einsatz kommen, bei denen R1 ein Derivat der Carboxylgruppe, wie z.B. eine Nitril- oder Estergruppe, darstellt, das durch Hydrolyse in die freie Carboxylgruppe überführt werden kann. Nach Abspaltung, z.B. durch Hydrierung der Schutzgruppen, erhält man die erfindungsgemäßen Komplexbildner, die gewünschtenfalls mit organischen und anorganischen Säuren und Basen umgesetzt werden können.

Ein weiterer Gegenstand sind Komplexe paramagnetischer Metallionen mit den erfindungsgemäßen Komplexbildnern und deren Salze, wobei Komplexe und deren Salze mit Eisen(III), Mangan(II) und Gadolinium(III) bevorzugt sind.

Als paramagnetische Metallionen kommen die Kationen der Übergangsmetalle der Ordnungszahlen 21 bis 29, 42 und 44 sowie die Kationen der Lanthaniden-Elemente der Ordnungszahlen 57 bis 70, wobei von den Lanthaniden-Elementen Gadolinium bevorzugt ist, in Frage.

Als Salze der erfindungsgemäßen Komplexe sind die Natrium- und N-Methylglucaminsalze bevorzugt.

Weitere Gegenstände der Erfindung sind Kontrastmittel für die MR-Diagnostik enthaltend erfindungsgemäße Komplexe und deren Salze sowie die Verwendung der erfindungsgemäßen Komplexe und deren Salze für die Herstellung von Kontrastmitteln für die MR-Diagnostik.

Bevorzugte Komplexe sind die Eisen(III)-Komplexe von
N,N'-Bis-(3-hydroxy-4-[1H]pyridon-2-yl-methyl)ethylendiamin-N,N'-diessigsäure,
N-(3-Hydroxy-4-[1H]pyridon-2-yl-methyl)ethylendiamin-N,N'-diessigsäure und N,N'-Bis-(3-hydroxy-4-[1H]-pyridon-2-yl-methyl)ethylendiamin,
die Gadolinium(III)-Komplexe von
N,N'-Bis-(3-hydroxy-4-[1H]pyridon-2-yl-methyl)ethylendiamin-N,N'-diessigsäure,
N,N''-Bis-(3-hydroxy-4-[1H]pyridon-2-yl-methyl)diethylentriamin-N,N',N''-triessigsäure und
N'-(3-Hydroxy-4-[1H]pyridon-2-methyl)-diethylentriamin-N,N,N''N''-tetraessigsäure sowie
der Mangan(II)-Komplex von N,N'-Bis-(3-hydroxy-4-[1H]pyridon-2-yl-methyl)-ethylendiamin-N,N'-diessigsäure.

Ein weiterer Gegenstand der Erfindung ist ein Verfahren zur Herstellung von Komplexen, dadurch gekennzeichnet, daß man Verbindungen der allgemeinen Formel I, worin R1, R2, R3, R4, R5, R6, R7, X, m, n, p und q die oben angegebene Bedeutungen haben, mit paramagnetischen Metallionen umsetzt und gewünschtenfalls die erhaltenen Komplexe mit Hilfe starker Basen in die Salze überführt.

Die erfindungsgemäßen Komplexe ergeben eine sehr gute Kontrasterhöhung bei der MR-Diagnostik und zeichnen sich gegenüber dem Stand der Technik durch zahlreiche Vorteile aus. Sie verursachen in den für die MR-Diagnostik relevanten Dosierungen keine Senkung des Blutdruckes und der Herzfrequenz. Sie werden unverändert und rasch renal ausgeschieden. Sie sind nicht hydrolyseempfindlich. Außerdem ergeben die erfindungsgemäßen Komplexbildner stabilere Komplexe als diejenigen nach dem Stand der Technik, die anstelle der Reste 3-Hydroxy-1-(R6)-4-[1H]-pyridon-2-yl Carboxylgruppen aufweisen. Ihre Herstellung ist einfach und wenig kostenaufwendig und es können Lösungen mit physiologischem pH-Wert hergestellt werden.

Erfindungsgemäße Kontrastmittel für die MR-Diagnostik enthalten einen oder mehrere der Komplexe paramagnetischer Metallionen mit Komplexbildnern der allgemeinen Formel I und gewünschtenfalls übliche Zusatzstoffe.

Die Herstellung der Komplexe erfolgt in an sich bekannter Weise dadurch, daß man Salze der paramagnetischen Metalle in Wasser und/oder einem Alkohol, wie Methanol, Ethanol usw., löst und mit der entsprechenden Menge des Komplexbildners gegebenenfalls unter Erhitzen auf 50 °C bis 120 °C solange rührt, bis die Umsetzung vollständig ist. Anstelle der Salze der paramagnetischen Metalle können auch Komplexe der paramagnetischen Metallionen eingesetzt werden, z.B. die entsprechenden Acetylacetonato-Komplexe, deren Liganden durch die erfindungsgemäßen Komplexbildner austauschbar sind. Wenn der gebildete Komplex im verwendeten Lösungsmittel unlöslich ist, kristallisiert er und kann abfiltriert werden. Wenn der Komplex im verwendeten Lösungsmittel löslich ist, kann er durch Eindampfen der Lösung zur Trockne isoliert oder durch Zugabe eines anderen organischen Lösungsmittels ausgefällt werden. Die erhaltene Komplexverbindung wird anschließend in Wasser gelöst oder suspendiert und mit der gewünschten anorganischen oder organischen Base bzw. Säure versetzt, bis der Neutralpunkt erreicht ist. Nach Filtration von ungelösten Anteilen wird die Lösung eingedampft und als Rückstand das gewünschte Komplexsalz erhalten oder das Komplexsalz aus wäßriger Lösung mit einem organischen Lösungsmittel ausgefällt. Die Salze der Komplexe werden beispielsweise durch Umsetzung mit starken Basen erhalten.

Die Herstellung der Komplexe kann in an sich bekannter Weise auch dadurch erfolgen, daß man eine wäßrige Lösung des Salzes des paramagnetischen Metalls zu einer Lösung der entsprechenden Menge des

Komplexbildners plus der entsprechenden Menge Base, z.B. Natriumhydroxid, zutropft und die wäßrige Lösung des Natriumsalzes des Komplexes steril filtiert.

Nach Sterilfiltration können die Komplexe in Form ihrer Salze durch Zugabe eines unpolaren, mit Wasser mischbaren Lösungsmittels (z.B. Aceton, Isopropanol) ausgefällt werden. Man erhält auf diese Weise die gewünschten Komplexsalze in fester Form frei von anorganischen Salzen.

Die Abtrennung von anorganischen Salzen kann auch durch Fällung der Komplexe durch Mineralsäure (z.B. wäßrige Salzsäure) bei pH 2,5 - 4 und Filtration oder in an sich bekannter Vorgehensweise über Ionenaustauscher geschehen.

Zur Überführung in die gewünschte, bei physiologischen pH-Werten lösliche Form (z.B. Natriumsalz oder Megluminsalz) wird die vom Ionenaustauscher eluierte wäßrige Lösung oder eine Suspension der gefällten Komplexverbindung in Wasser mit der entsprechenden Menge an starker Base (z.B. Natronlauge oder N-Methylglucamin) versetzt. Man erhält auf diese Weise Lösungen der gewünschten Komplexsalze frei von anorganischen Säuren.

Die Herstellung der neuen Kontrastmittel erfolgt auf an sich bekannte Weise, indem man die Komplexe und/oder deren Salze in Wasser oder physiologischer Salzlösung auflöst und gewünschtenfalls in der Galenik übliche Zusätze, wie beispielsweise physiologisch verträgliche Pufferlösungen (z.B. Natriumdihydrogenphosphatlösung), Albumin und dergleichen, zusetzt und die Lösung sterilisiert.

Die Lösungen können als solche in Ampullen abgefüllt werden oder zu einem löslichen Pulver gefriergetrocknet werden. Die wäßrigen Lösungen werden auf einen pH-Wert mit ausreichender lokaler Gewebeverträglichkeit eingestellt, beispielsweise auf einen pH-Wert von 7 bis 9. Die wäßrigen Lösungen werden oral oder parenteral, insbesondere intravasal verabreicht. Zur MR-Diagnostik beim Menschen werden wäßrige Lösungen verwendet, die den paramagnetischen Komplex in einer Konzentration von 30 bis 500 mMol/Liter enthalten. Pro Anwendung werden ungefähr 1 bis 300 µMol des Komplexes pro kg Körpergewicht verabreicht. Für einen Erwachsenen erweist sich für eine i.v.-Anwendung eine Dosis von 1 bis 100 mMol als angebracht.

## Beispiele

1. N,N'-Bis-(3-hydroxy-4-[1H]pyridon-2-yl-methyl)ethylendiamin-diessigsäure-dihydrochlorid

a) N,N'-Bis-(3-hydroxy-4-[1H]pyridon-2-yl-methyl)ethylendiamin-diessigsäure-dihydrochlorid

2,0 g N,N'-Bis[(3,4-bis-benzyloxy-2-pyridyl)methyl]ethylendiamin-diessigsäure werden unter Zusatz von 2,5 ml 2N Salzsäure in 50 ml Methanol gelöst und nach Zugabe von 0,1 g 10 %igem Palladium auf Aktivkohle-Katalysator an einer Umlaufhydrierapparatur bei ca. 0,1 bar Überdruck Wasserstoff hydriert. Nach 2 Stunden wird vom Katalysator filtriert, eingeengt und durch Zugabe von Diethylether kristallisiert.

Man erhält 1,28 g (95 % der Theorie) der Titelverbindung als farbloses Kristallisat (Dihydrat; Zersetzung ab 75 °C).

| Analyse: | | | | |
|---|---|---|---|---|
| berechnet: | C 40,83 | H 5,33 | N 10,58 | Cl 13,40 |
| gefunden: | C 41,08 | H 5,16 | N 10,30 | Cl 13,21 |

b) N,N'-Bis-[(3,4-bis-benzyloxy-2-pyridyl)methyl]ethylendiamin-diessigsäure

4 g N,N'-Bis-[(3,4-bis-benzyloxy-2-pyridyl)methyl]ethylendiamin werden in 50 ml Ethanol und 10 ml Wasser gelöst und bei 60 °C eine Mischung aus 1,83 g Bromessigsäure und 13,2 ml 1N Natronlauge zugetropft. Dabei hält man den pH-Wert der Reaktionsmischung mit Natronlauge zwischen 10 bis 11. Nach 4 Stunden wird überschüssiges Ethanol im Vakuum abdestilliert, der pH-Wert mit Salzsäure auf 3 gestellt und mehrmals mit Dichlormethan extrahiert. Die vereinigten organischen Phasen werden mit Wasser gewaschen, getrocknet (Magnesiumsulfat) und am Rotationsverdampfer vollständig eingeengt. Der amorphe Rückstand wird aus heißem Aceton zur Kristallisation gebracht. Man erhält 3,2 g (68 % der Theorie) der Titelverbindung als farblosen Feststoff vom F. 159-161 °C.

c) N,N'-Bis-[(3,4-bis-benzyloxy-2-pyridyl)methyl]ethylendiamin

7,5 g N,N'-Bis-[(3,4-bis-benzyloxy-2-pyridyl)methylen]ethylendiamin, gelöst in 100 ml Methanol werden portionsweise mit 1,1 g Natriumborhydrid versetzt und 18 Stunden gerührt. Das Lösungsmittel wird im Vakuum vollständig eingeengt, der Rückstand in Dichlormethan aufgenommen, das unlösliche anorganische Salz filtriert, die organische Phase mit Wasser neutral gewaschen, getrocknet (Kaliumcarbonat) und vollständig eingeengt. Der Rückstand wird aus Ethylacetat/Cyclohexan zur Kristallisation gebracht. Man erhält 6,1 g (81 % der Theorie) der Titelverbindung vom F. 61-62° C.

d) N,N'-Bis-[(3,4-bis-benzyloxy-2-pyridyl)methylen]ethylendiamin

10 g 3,4-Bis-benzyloxy-pyridin-2-aldehyd werden in 250 ml Dioxan mit 1,05 ml (0,5 Äquivalente) Ethylendiamin kondensiert, wobei man entstehendes Reaktionswasser azeotrop entfernt. Nach 4 Stunden wird das Lösungsmittel im Vakuum abdestilliert, der Rückstand in Diisopropylether bei 60° C gelöst und auf Raumtemperatur abgekühlt. Das kristalline Produkt wird filtriert und getrocknet. Man erhält 3,4 g (91 % der Theorie) der Titelverbindung vom F. 124-126° C.

e) N,N'-Bis-[(3,4-bis-benzyloxy-2-pyridyl)methyl]ethylendiamin-diessigsäure

Die Verbindung 1b) erhält man auch durch Umsetzung von N,N'-Bis-[(3,4-bis-benzyloxy-2-pyridyl)-methyl]N,N'-bis-cyanomethyl-ethylendiamin mit überschüssiger KOH in Ethylenglycol unter Rückfluß nach Aufarbeitung, wie in Beispiel 1b) beschrieben.

f) N,N'-Bis-[(3,4-bis-benzyloxy-2-pyridyl)methyl]-N,N'-bis-cyanomethyl-ethylendiamin

Zu 0,5 g N,N'-Bis-[(3,4-bis-benzoyloxy-2-pyridyl)methyl]ethylendiamin und 2 Äquivalenten 0,1N HCl werden 0,15 g (4 Äquivalente) Natriumcyanid in 10 ml Wasser und 0,09 g Formaldehydlösung (35 %ig; 4 Äquivalente) zugegeben. Die entstandene Suspension wird 36 Stunden kräftig gerührt. Nach Filtration wird der Feststoff erneut in 10 ml Wasser aufgerührt, filtriert, gut mit Wasser nachgewaschen und im Vakuum bei 58° C getrocknet. Man erhält 0,45 g (81 % d.Th.) der Titelverbindung vom F. 103 - 105° C.

2. N,N'-Bis-(3-hydroxy-4-[1H]pyridon-2-yl-methyl)ethylendiamin-N,N'-diessigsäure-dihydrobromid

a) N,N'-Bis-(3-hydroxy-4-[1H]pyridon-2-yl-methyl)ethylendiamin-N,N'-diessigsäure-dihydrobromid

Die Titelverbindung erhält man als Dihydrobromid auch durch Umsetzung von N,N'-Bis-[(3,4-dimethoxy-2-pyridyl)methyl]ethylendiamin-N,N'-diethylacetat mit 48 %iger Bromwasserstoffsäure. Nach 3 Stunden Reaktion bei 100° C wird vollständig eingedampft, der Rückstand in Wasser gelöst, mit Aktivkohle geklärt. Nach erneutem vollständigen Eindampfen und Umkristallisieren aus Ethanol/Diisopropylether erhält man die Titelverbindung als hygroskopisches Dihydrobromid.

b) N,N'-Bis-[(3,4-dimethoxy-2-pyridyl)methyl]ethylendiamin-N,N'-bis-ethylacetat

5,0 g N,N'-Bis-[(3,4-dimethoxy-2-pyridyl)methyl]ethylendiamin werden mit 5,07 g Bromessigsäureethylester in 100 ml Toluol und 20 ml Wasser unter Zusatz von 6,9 g Kaliumhydrogencarbonat und 0,2 g Kaliumiodid 1 Stunde auf Rückflußtemperatur erhitzt. Nach dem Abkühlen werden die Phasen getrennt. Die organische Phase wird mit Wasser gewaschen, getrocknet (Kaliumcarbonat) und anschließend eingeengt. Der Rückstand wird über Kieselgel chromatographiert. Man erhält 3,7 g (50 % der Theorie) der Titelverbindung als farbloses Öl.

c) N,N'-Bis-[(3,4-dimethoxy-2-pyridyl)methyl]ethylendiamin

Nach der in Beispiel 1c) beschriebenen Arbeitsweise erhält man durch Umsetzung von 27 g N,N'-Bis-[-(3,4-dimethoxy-2-pyridyl)methylen]ethylendiamin mit 10,8 g Natriumborhydrid in 300 ml Methanol nach Kristallisation aus Diisopropylether 20,8 g (76 % der Theorie) der Titelverbindung vom F. 69-70° C.

d) N,N'-Bis-[(3,4-dimethoxy-2-pyridyl)methylen]ethylendiamin

Nach der in Beispiel 1d) angegebenen Arbeitsweise erhält man durch Umsetzung von 32 g 3,4-Dimethoxy-pyridin-2-aldehyd mit 6,40 ml Ethylendiamin in 300 ml Dioxan nach Kristallisation aus Diisopropylether 27,8 g (81 % der Theorie) N,N'-Bis-[(3,4-dimethoxy-2-pyridyl)methylen]ethylendiamin vom F. 132-134° C.

3. N,N''-Bis-(3-hydroxy-4-[1H]pyridon-2-yl-methyl)diethylentriamin-N,N',N''-triessigsäure

a) N,N''-Bis-(3-hydroxy-4-[1H]pyridon-2-yl-methyl)diethylentriamin-N,N',N''-triessigsäure

N,N''-Bis-[(3,4-bis-benzyloxy-2-pyridyl)methyl]diethylentriamin-N,N',N''-triessigsäure-trinatriumsalz werden, wie in Beispiel 1a) beschrieben, unter Zusatz von 6 Äquivalenten 4N Salzsäure hydriert. Nach beendeter Hydrierung wird vom Katalysator filtriert, eingeengt und mit Diisopropylether verrieben. Man erhält die Titelverbindung als farblosen amorphen Feststoff im Gemisch mit Natriumchlorid.

b) Trinatrium-N,N''-bis-[(3,4-bis-benzyloxy-2-pyridyl)methyl]diethylentriamin-N,N',N''-triacetat

9,5 g N,N''-Bis-[(3,4-bis-benzyloxy-2-pyridyl)methyl]diethylentriamin werden mit der in Beispiel 1b) beschriebenen Arbeitsweise mit 6,55 g Bromessigsäure (3 Äquivalente) umgesetzt. Nach Abdestillieren des Ethanols wird der Rückstand in Wasser aufgenommen, ein pH-Wert von 13 bis 14 eingestellt und dreimal mit 50 ml Dichlormethan extrahiert. Die vereinigten organischen Phasen werden eingeengt und der Rückstand durch Verreiben mit Diisopropylether kristallisiert. Man erhält die Titelverbindung als farblosen Feststoff vom F. >250° C (Zers.).

c) N,N''-Bis-[(3,4-bis-benzyloxy-2-pyridyl)methyl]diethylentriamin

5 g 3,4-Bis-benzyloxy-pyridin-2-aldehyd werden nach der in Beispiel 1d) beschriebenen Arbeitsweise mit 0,81 g (0,5 Äquivalente) Diethylentriamin in 50 ml Dioxan umgesetzt und das ölige Reaktionsprodukt N,N''-Bis-[(3,4-bis-benzyloxy-2-pyridyl)methylen]diethylentriamin in 50 ml Methanol mit 0,88 g Natriumborhydrid wie in Beispiel 1c) beschrieben umgesetzt. Aus Ethylacetat/Cyclohexan erhält man 3,1 g (55 % der Theorie) der Titelverbindung vom F. 55 - 57° C als Monohydrat.

4. 3,4-Bis-benzyloxy-pyridin-2-aldehyd

a) 3,4-Bis-benzyloxy-pyridin-2-aldehyd

50 g 3,4-Bis-benzyloxy-2-hydroxymethyl-pyridin werden in 500 ml Dioxan gelöst und mit 50 g aktiven Mangandioxid versetzt. Das Reaktionswasser wird durch 20-stündige azeotrope Destillation bei Normaldruck entfernt. Nach Filtration engt man im Vakuum auf ca. 100 ml ein und versetzt bei 70 bis 80° C bis zur Trübung mit Diisopropylether. Nach Abkühlung wird filtriert. Man erhält 46,5 g (94 % der Theorie) der Titelverbindung vom F. 98-100° C.

b) 3,4-Bis-benzyloxy-2-hydroxymethylpyridin

In 500 ml Acetanhydrid trägt man bei 65° C portionsweise 187 g 3,4-Bis-benzyloxy-2-methyl-1-oxy-pyridin innerhalb von 2 Stunden ein. Man rührt weitere 2 Stunden bei 65-80° C und entfernt anschließend überschüssiges Acetanhydrid bei 10 mbar am Rotationsverdampfer. Der Rückstand wird in Methanol aufgenommen, mit 300 ml 2N Natronlauge auf pH 11 bis 12 gestellt und 2 Stunden bei 80° C gerührt. Dabei wird das Zwischenprodukt 2-Acetoxymethyl-3,4-bis-benzyloxypyridin zum 2-Hydroxymethyl-3,4-bis-benzyloxypyridin hydrolysiert. Nach Abkühlung extrahiert man zweimal mit je 300 ml Dichlormethan, wäscht die vereinigten organischen Phasen mit Wasser, trocknet über Natriumsulfat, engt ein und kristallisiert mit Diisopropylether. Man erhält 3,4-Bis-benzyloxy-2-hydroxymethylpyridin vom F. 86-88° C.

c) 3,4-Bis-benzyloxy-2-methyl-1-oxy-pyridin

In eine Lösung von Natriumbenzylalkoholat, hergestellt aus 46 g Natriumhydrid (80 %ig) und 160 ml Benzylalkohol in 800 ml Dimethylsulfoxid, tropft man unter Kühlung eine Lösung von 192 g 3-Benzyloxy-4-chlor-2-methyl-1-oxy-pyridin in 500 ml Dimethylsulfoxid und 250 ml Tetrahydrofuran in der Weise ein, daß die Temperatur 25° C nicht übersteigt. Nach dreistündigem Rühren bei 20° C tropft man vorsichtig bis zur Beendigung der Gasentwicklung Wasser zu. Anschließend werden zusätzlich 10 l Wasser zugegeben und 20 Stunden gerührt. Der kristalline Niederschlag wird über eine Nutsche filtriert, mit Wasser gewaschen, in

500 ml Dichlormethan gelöst und über Natriumsulfat getrocknet. Das Lösungsmittel wird am Rotationsverdampfer unter Vakuum entfernt und der Rückstand aus Petrolether 50/70 kristallisiert. Man erhält 135 g (55 % der Theorie) 3,4-Bis-benzyloxy-2-methyl-1-oxy-pyridin als beigefarbenes Kristallisat vom F. 118-120 °C.

d) 3-Benzyloxy-4-chlor-2-methyl-1-oxy-pyridin

289 g (1,24 Mol) 3-Benzyloxy-4-chlor-2-methylpyridin werden in 1400 ml Eisessig gelöst, auf 80 °C erwärmt, 3 ml konz. Schwefelsäure zugesetzt und 500 ml 30 %ige Wasserstoffperoxidlösung innerhalb einer Stunde zugetropft. Man rührt 20 Stunden bei 80 °C, kühlt ab und engt die ca. 50 °C warme Mischung im Vakuum weitgehend ein. Der Rückstand wird in Dichlormethan gelöst, durch vorsichtige Zugabe von Braunstein noch vorhandene Peroxide zerstört, über Celit filtriert und mehrfach unter Zusatz von Toluol zur Trockene eingeengt. Der Rückstand, 292 g (95 % der Theorie) öliges blaßgelbes 3-Benzyloxy-4-chlor-2-methyl-1-oxy-pyridin, ist für eine Weiterumsetzung von ausreichender Reinheit. Bei längerem Stehen kristallisiert das Produkt.

Zum Zwecke der Charakterisierung wurde eine Probe aus Diethylether kristallisiert. Man erhält 3-Benzyloxy-4-chlor-2-methyl-oxy-pyridin in Form gelber Prismen vom F. 82-84 °C.

e) 3-Benzyloxy-4-chlor-2-methylpyridin

Zu 1500 ml Phosphoroxytrichlorid dosiert man innerhalb einer Stunde unter Rühren 150 g (0,7 Mol) 3-Benzyloxy-2-methyl-4[1H]pyridon zu, wobei die Innentemperatur bis 32 °C ansteigt. Anschließend rührt man 30 Stunden bei 50 °C, destilliert überschüssiges Phosphoroxytrichlorid im Vakuum ab und läßt den Rückstand langsam in eine eiskalte Lösung von 280 g Natriumhydroxid in 1,5 l Wasser unter gutem Rühren einlaufen. Man extrahiert dreimal mit je 500 ml Toluol, wäscht die vereinigten organischen Phasen mit Wasser, klärt mit Tonsil, Celit und Aktivkohle und engt vollständig ein. Man erhält 147 g (90 % der Theorie) 3-Benzyloxy-4-chlor-2-methylpyridin als blaßgelbes Öl, das ohne weitere Reinigung weiter umgesetzt wurde.

Zur Charakterisierung wurde ein kleiner Teil in etherischer Lösung mit Salzsäuregas in das Hydrochlorid übergeführt. Man erhält ein farbloses Kristallisat vom F. 150-151 °C.

f) 3-Benzyloxy-2-methyl-4-[1H]pyridon

348 g Maltol werden in 13 l Aceton gelöst, 367 g Benzylchlorid und 2 g Kaliumiodid zugesetzt und auf Rückflußtemperatur erwärmt. Anschließend dosiert man unter intensivem Rühren innerhalb von 5 Stunden 572 g gemahlenes Kaliumcarbonat zu und rührt weitere 20 Stunden unter Rückfluß. Nach Filtration von anorganischen Salzen wird im Vakuum vollständig eingeengt und der hellgelbe, ölige Rückstand (3-Benzyloxy-2-methyl-4-pyron) mit 2,7 l 25 %iger Ammoniaklösung versetzt. Man erwärmt vorsichtig innerhalb von 3 Stunden auf 60 °C und dosiert weitere 2,8 l Ammoniaklösung innerhalb von 3 Stunden zu. Anschließend rührt man 20 Stunden bei 80 °C, kühlt ab, extrahiert viermal mit je 2 l Dichlormethan, wäscht die vereinigten organischen Phasen mit 1 l Wasser, engt am Rotationsverdampfer weitgehend ein und kristallisiert durch Zugabe von 2 l Diisopropylether. Nach Abkühlung auf 5 °C wird über eine Nutsche filtriert, mit Diisopropylether gewaschen und im Vakuumtrockenschrank bei 40 °C bis zur Gewichtskonstanz getrocknet. Man erhält 421 g (71 % der Theorie) 3-Benzyloxy-2-methyl-4[1H]pyridon als elfenbeinfarbenes Kristallisat vom F. 168-170 °C.

5. 3,4-Dimethoxypyridin-2-aldehyd

Nach der in Beispiel 4 a) beschriebenen Arbeitsweise erhält man aus 100 g 2-Hydroxymethyl-3,4-dimethoxypyridin mit 150 g aktivem Mangandioxid nach Kristallisation aus Diisopropylether 75,1 g (76 % der Theorie) der Titelverbindung vom F. 64-66 °C.

6. N-(3-Hydroxy-4-[1H]pyridon-2-yl-methyl)ethylendiamin-N,N'-diessigsäure-dihydrochlorid

a) N-(3-Hydroxy-4-[1H]pyridon-2-yl-methyl)ethylendiamin-N,N'-diessigsäure-dihydrochlorid

N-[(3,4-Bis-benzyloxy-2-pyridyl)methyl]ethylendiamin-N,N'-diessigsäure wird unter Zusatz von 2,1 Äquivalenten Salzsäure, wie unter Beispiel 1a) beschrieben, hydriert. Man erhält die Titelverbindung als farblosen Feststoff vom F. 226 - 228 °C.

b) N-[(3,4-Bis-benzyloxy-2-pyridyl)methyl]ethylendiamin-N,N'-diessigsäure

2,0 g (11,4 mMol) Ethylendiamin-N,N'-diessigsäure, gelöst in 20 ml 4N Natronlauge, werden zu 3,4-Bisbenzyloxy-2-chlormethylpyridiniumchlorid (4,27 g, 11,4 mMol, erhalten durch Umsetzung von 3,4-Bisbenzyloxy-2-hydroxymethylpyridin mit Thionylchlorid in Dichlormethan nach der üblichen Verfahrensweise, F. 141 - 142°C), gelöst in 40 ml Ethanol, innerhalb von 4 Stunden bei 60°C zudosiert. Nach weiteren 2 Stunden bei 60°C wird eingeengt, der Rückstand mit 100 ml Wasser versetzt, mit Salzsäure auf pH 3 gestellt und mehrfach mit Methylenchlorid extrahiert. Man erhält als Nebenprodukt nach Einengen der organischen Phase das Produkt aus Beispiel 1b). Aus der Wasserphase kristallisiert ein farbloser Feststoff. Man filtriert und trocknet bis zur Gewichtskonstanz. Man erhält die Titelverbindung vom F. 92 - 93°C.

7. N,N'-Bis-(3-hydroxy-4-[1H]-pyridon-2-yl-methyl)ethylendiamin-monoessigsäure-dihydrochlorid

a) N,N'-Bis-(3-hydroxy-4-[1H]-pyridon-2-yl-methyl)ethylendiamin-monoessigsäure-dihydrochlorid

Nach der in Beispiel 1a) beschriebenen Arbeitsweise erhält man aus 0,18 g N,N'-Bis-[(3,4-bis-benzyloxy-2-pyridyl)methyl]ethylendiamin-monoessigsäure nach Kristallisation aus Methanol/Aceton 0,12 g der Titelverbindung in Form farbloser Kristalle vom F. 217 - 219°C (enthält Kristallwasser).

b) N,N'-Bis-[(3,4-bis-benzyloxy-2-pyridyl)methyl]ethylendiamin-monoessigsäure-dihydrochorid

1,0 g (1,5 mmol) N,N'-Bis-[(3,4-bis-benzyloxy-2-pyridyl)methyl]ethylendiamin werden nach der in Beispiel 1b) beschriebenen Arbeitsweise mit 0,25 g (1,8 mmol) Bromessigsäure umgesetzt und aufgearbeitet. Der amorphe Rückstand wird in Aceton gelöst und mit 2 Äquivalenten konzentrierter Salzsäure versetzt. Der ausgefallene Feststoff wird abgesaugt und zweimal aus Aceton/Methanol umkristallisiert. Man erhält 0,8 g der Titelverbindung vom F. 179 - 180°C.

8. N,N'-Bis-(3-hydroxy-4-[1H]-pyridon-2-yl-methyl)ethylendiamin-tetrahydrochlorid

Nach der in Beispiel 1a) beschriebenen Arbeitsweise enthält man durch Hydrierung von 1,0 g N,N'-Bis-[(3,4-bis-benzyloxy-2-pyridyl)methyl]ethylendiamin in 60 ml Methanol in Gegenwart von 0,1 g Palladium/Kohle und 1,5 ml (4 Äquivalente) 4N Salzsäure 0,6 g (89 % d. Th.) der Titelverbindung vom F. 166°C (Zers.).

9. Gadolinium(III)-mononatrium-N,N'-bis-(4-[1H]pyridon-3-olato-2-yl-methyl)-ethylendiamin-diacetat-tetrahydrat

0,53 g (1 mmol) N,N'-Bis-(3-hydroxy-4-[1H]-pyridon-2-yl-methyl)ethylendiamin-diessigäure-dihydrochlorid werden in 5 ml Wasser gelöst. Nach Zugabe von 1 Äquivalent Gadoliniumtrichlorid x 6 $H_2O$ wird mit 2N NaOH (4 Äquivalente) auf pH 3 gestellt und 5 Stunden gerührt. Man filtriert vom ausgefallenen Feststoff, wäscht mit Wasser nach und trocknet bei 50°C.

Man erhält 0,48 g der Titelverbindung vom F. >300°C.

| Analyse: | | | |
|---|---|---|---|
| berechnet: | C 32,24 | H 3,91 | N 8,35 |
| gefunden: | C 32,87 | H 3,82 | N 8,50 |

## Patentansprüche

**1.** Komplexbildner der allgemeinen Formel I, worin

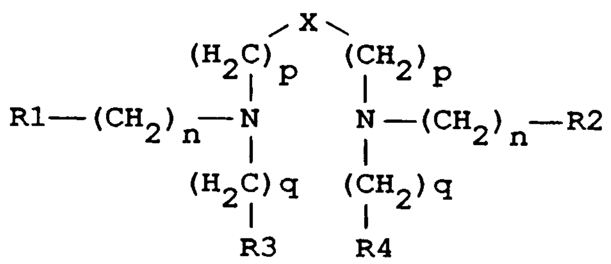

(I),

R1, R2, R3 und R4 gleich oder verschieden sind und für H, COOH, C(O)NHR5, wobei
R5 H und C1-C4-Alkyl bedeutet,
$PO_3H_2$, $SO_3H$ und den Rest 3-Hydroxy-1-(R6)-4-[1H]pyridon-2-yl, wobei
R6 H und C1-C4-Alkyl bedeutet,
stehen, mit der Maßgabe, daß mindestens einer der Reste R1, R2, R3 und R4 die Bedeutung 3-Hydroxy-1-(R6)-4-[1H]pyridon-2-yl mit R6 = H und C1-C4-Alkyl hat,
X für eine Einfachbindung, $(CH_2)_m$ und NR7, wobei
R7 H, C1-C4-Alkyl und $(CH_2)_n$R1 und
m 1 und 2 bedeuten,
n,q für ganze Zahlen von 0 bis 2, wobei n und q gleich oder verschieden sind und deren Summe mindestens 1 beträgt, und
p für eine ganze Zahl von 1 bis 3, aber nicht für 1, wenn X die Bedeutung NR7 hat,

stehen und deren Salze mit organischen und anorganischen Basen und Säuren.

**2.** Komplexbildner nach Anspruch 1, dadurch gekennzeichnet, daß in der allgemeinen Formel I
R1, R2, R3 und R4 gleich oder verschieden sind und für H, COOH, C(O)NHR5, wobei
R5 H, Methyl und Ethyl bedeutet, und den Rest 3-Hydroxy-4-[1H]-pyridon-2-yl stehen, mit der Maßgabe, daß mindestens einer der Reste R1, R2, R3 und R4 für die Bedeutung 3-Hydroxy-4-[1H]pyridon-2-yl steht,
X für eine Einfachbindung, $CH_2$ und NR7, wobei
R7 H und $(CH_2)_n$R1 bedeutet,
n,q für 1 und
p für 1 und 2, aber nicht für 1, wenn X die Bedeutung NR7 hat, und nicht für 2, wenn X die Bedeutung Einfachbindung hat,

stehen, und deren Salze mit organischen und anorganischen Basen und Säuren.

**3.** Komplexbildner nach Anspruch 1, dadurch gekennzeichnet, daß in der allgemeinen Formel I
R1, R2, R3 und R4 gleich oder verschieden sind und für H, COOH und den Rest 3-Hydroxy-4-[1H]pyridon-2-yl stehen, mit der Maßgabe, daß mindestens einer der Reste R1, R2, R3 und R4 für den Rest 3-Hydroxy-4-[1H]pyridon-2-yl steht,
X für eine Einfachbindung,
n für 1,
q für 0 und 1 und
p für 1

stehen, und deren Salze mit organischen und anorganischen Basen und Säuren.

**4.** Komplexbildner nach Anspruch 1, dadurch gekennzeichnet, daß in der allgemeinen Formel I
R1, R2, R3 und R4 gleich oder verschieden sind und für H, COOH und den Rest 3-Hydroxy-4-[1H]pyridon-2-yl stehen, mit der Maßgabe, daß mindestens einer der Reste R1, R2, R3 und R4 für den Rest 3-Hydroxy-4-[1H]pyridon-2-yl steht,
X für NR7, wobei R7 $(CH_2)_n$R1 bedeutet,

| | |
|---|---|
| n | für 1, |
| q | für 0 und 1 und |
| p | für 2 |

stehen, und deren Salze mit organischen und anorganischen Basen und Säuren.

**5.** N,N'-Bis-(3-hydroxy-4-[1H]pyridon-2-yl-methyl)ethylendiamin-N,N'-diessigsäure, N,N'-Bis-(3-hydroxy-4-[1H]-pyridon-2-yl-methyl)ethylendiamin-mono-essigsäure, N,N'-Bis-(3-hydroxy-4-[1H]-pyridon-2-yl-methyl)ethylendiamin, N,N''-Bis-(3-hydroxy-4-[1H]pyridon-2-yl-methyl)diethylentriamin-N,N',N''-triessigsäure, N-(3-Hydroxy-4-[1H]pyridon-2-yl-methyl)ethylendiamin-N,N',N'-triessigsäure, N'-(3-Hydroxy-4-[1H]-pyridon-2-yl-methyl)diethylentriamin-N,N,N'',N''-tetraessigsäure und N-(3-Hydroxy-4-[1H]pyridon-2-yl-methyl)ethylendiamin-N,N'-diessigsäure und deren Salze mit organischen und anorganischen Basen und Säuren.

**6.** Komplexbildner nach Anspruch 1, dadurch gekennzeichnet, daß ein oder zwei der Reste R1, R2, R3 und R4 die Bedeutung H und ein oder zwei der Reste R1, R2, R3 und R4 die Bedeutung 3-Hydroxy-4-[1H]pyridon-2-yl haben, und deren Salze mit organischen und anorganischen Basen und Säuren.

**7.** Komplexe paramagnetischer Metallionen mit Komplexbildnern nach den Ansprüchen 1 bis 6 und deren Salze.

**8.** Komplexe nach Anspruch 7, dadurch gekennzeichnet, daß die paramagnetischen Metallionen Eisen(III), Mangan(II) und Gadolinium(III) sind.

**9.** Komplexe nach Anspruch 7, dadurch gekennzeichnet, daß sie als Natrium- oder N-Methylglucaminsalze vorliegen.

**10.** Kontrastmittel für die MR-Diagnostik enthaltend Komplexe nach den Ansprüchen 7, 8 und 9.

**11.** Verfahren zur Herstellung von Komplexen, dadurch gekennzeichnet, daß man Verbindungen der allgemeinen Formel I, worin R1, R2, R3, R4, R5, R6, R7, X, m, n, p und q die in Anspruch 1 angegebenen Bedeutungen haben, mit paramagnetischen Metallionen umsetzt und gewünschtenfalls die erhaltenen Komplexe mit Hilfe starker Basen in die Salze überführt.

**12.** Verfahren zur Herstellung der Komplexbildner der allgemeinen Formel I, dadurch gekennzeichnet, daß man eine Verbindung der allgemeinen Formel I, bei der die 3-Hydroxy-4-[1H]pyridon-2-yl-Reste durch 3,4-Dihydroxypyridin-2-yl-Reste, deren Hydroxygruppen durch für Hydroxygruppen übliche Schutzgruppen, vorzugsweise Benzyl und Methyl, geschützt sind, ersetzt sind, unter die Schutzgruppen abspaltenden Bedingungen umsetzt und gewünschtenfalls die 3-Hydroxy-4-[1H]pyridon-2-yl-Reste in 1-Stellung C1-C4-alkyliert.

Europäisches Patentamt

# EUROPÄISCHER RECHERCHENBERICHT

Nummer der Anmeldung

EP 92 10 1854

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| A | EP-A-0 138 421 (NATIONAL RESEARCH DEVELOPMENT)(24-04-1985)<br>--- | | C 07 D 213/69<br>A 61 K 31/44 |
| A | EP-A-0 292 761 (SALUTAR INC.)(30-11-1988)<br>----- | | |
| | | | **RECHERCHIERTE SACHGEBIETE (Int. Cl.5)** |
| | | | C 07 D 213/00 |

Der vorliegende Recherchenbericht wurde für alle Patentansprüche erstellt

| Recherchenort | Abschlußdatum der Recherche | Prüfer |
|---|---|---|
| DEN HAAG | 16-03-1992 | DE JONG B.S. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)